# EUROPEAN PATENT SPECIFICATION

(11) **EP 1 726 663 B1**
(45) Date of publication and mention of the grant of the patent: **18.08.2010**
(21) Application number: 05727696.6
(22) Date of filing: 18.03.2005
(51) Int. Cl.: C12Q 1/68, C12N 15/09, G01N 21/78, G01N 33/53, G01N 33/566

(54) **METHOD OF DETECTING GENE MUTATION AND KIT FOR DETECTING GENE MUTATION**
VERFAHREN ZUM NACHWEIS VON GENMUTATION UND KIT ZUM NACHWEIS VON GENMUTATION
PROCEDE DE DETECTION DE LA MUTATION D'UN GENE ET KIT SERVANT A DETECTER LA MUTATION D'UN GENE

(30) Priority: 19.03.2004 JP 2004080703
(43) Date of publication of application: 29.11.2006
(73) Proprietor: Japan Science and Technology Agency, Kawaguchi-shi, Saitama 332-0012 (JP)
(72) Inventor: TERAMAE, Norio, Sendai-shi, Miyagi 9800861 (JP); NISHIZAWA, Seiichi, Sendai-shi, Miyagi 9810952 (JP); YOSHIMOTO, Keitaro, Wako-shi, Saitama 3510111 (JP); SEINO, Takehiro, Sendai-shi, Miyagi 9820833 (JP); SATO, Fuyuki, Sendai-shi, Miyagi 9810967 (JP)
(74) Representative: Williams, Aylsa
(86) International application number: PCT/JP2005/006405
(87) International publication number: WO 2005/090604

(56) References cited:
- JP-A- 2001 089 478
- JP-A- 2001 149 096
- JP-A- 2004 261 068
- OKAMOTO AKIMITSU ET AL: "Design of base-discriminating fluorescent nucleoside and its application to T/C SNP typing." JOURNAL OF THE AMERICAN CHEMICAL SOCIETY, vol. 125, no. 31, 6 August 2003 (2003-08-06), pages 9296-9297, XP002453214 ISSN: 0002-7863
- OKAMOTO A ET AL: "Synthesis and properties of a novel fluorescent nucleobase, naphthopyridopyrimidine" TETRAHEDRON LETTERS, ELSEVIER, AMSTERDAM, NL, vol. 44, no. 36, 1 September 2003 (2003-09-01), pages 6871-6874, XP004444422 ISSN: 0040-4039
- YOSHIMOTO K ET AL: "Use of a basic site-containing DNA strands for Nucleobase recognition in water" JOURNAL OF THE AMERICAN CHEMICAL SOCIETY, AMERICAN CHEMICAL SOCIETY, WASHINGTON, DC, US, vol. 125, no. 30, 30 July 2003 (2003-07-30), pages 8982-8983, XP002989868 ISSN: 0002-7863
- OKAMOTO AKIMITSU ET AL: "Clear distinction of purine bases on the complementary strand by a fluorescence change of a novel fluorescent nucleoside." JOURNAL OF THE AMERICAN CHEMICAL SOCIETY, vol. 125, no. 17, 30 April 2003 (2003-04-30), pages 4972-4973, XP002453215 ISSN: 0002-7863
- NISHIZAWA SEIICHI ET AL: "Fluorescence detection of cytosine/guanine transversion based on a hydrogen bond forming ligand" TALANTA, vol. 63, no. 1, 10 May 2004 (2004-05-10), pages 175-179, XP002453216 ISSN: 0039-9140
- SANDO S ET AL: "New method for scanning of single-nucleotide polymorphisms (SNPs): recognition of guanine-guanine mismatches by dimeric naphthyridine." NUCLEIC ACIDS SYMPOSIUM SERIES 2000, no. 44, 2000, pages 119-120, XP002453217 ISSN: 0261-3166
- YOSHIMOTO K. ET AL: 'Use of a basic site-containing DNA strands for Nucleobase recognition in water.' J.AM.CHEM.SOC. vol. 125, 2003, pages 8982 - 8983, XP002989868
- NAKATANI K. ET AL: 'Scanning of guanine-guanine mismatches in DNA by synthetic ligands using surface plasmon resonance.' NAT.BIOTECHNOL. vol. 19, no. 1, January 2001, pages 51 - 55, XP001004477
- NAKATANI K. ET AL: 'Improved selectivity for the binding of naphthyridine dimer to guanine-guanine mismatch.' BIOORG.MED.CHEM. vol. 9, no. 9, September 2001, pages 2381 - 5, XP002982569
- NAKATANI K. ET AL: 'Recognition of Guadiane-Guadiane mismatches by the dimeric form of 2-amino-1,8-naphthyridine.' J.AM.CHEM.SOC. vol. 123, 2001, pages 12650 - 12657, XP002989869
- KREUZER K-A; ET AL: "Preexistence and evolution of imatinib mesylate-resistant clones in chronic myelogenous leukemia detected by a PNA-based PCR clamping technique" ANNALS OF HEMATOLOGY, vol. 85, no. 2, 5 January 2003 (2003-01-05), pages 284-289, XP002464825

## Description

### Technical Field

The present invention relates to a method for detecting a gene mutation and a kit for detecting a gene mutation using the method that are especially useful in the field of bio-informatics (life information science), and more particularly to a method for detecting a gene mutation and a kit for detecting a gene mutation by which single nucleotide substitution in a gene arrangement is simply and rapidly detected.

This application claims a priority based on Japanese Patent Application No. 2004-080703 filed on March 19, 2004 in Japan that is applied to this application with reference thereto.

### Background Art

One of the targets of a study after a human genome arrangement is decoded nowadays resides in an identification of a gene, an analysis of a function and the variety of genes for determining a individual difference influenced by the expression or the function of the gene. Here, the solid difference of the gene caused from the difference of single nucleotide in a nucleobase sequence is referred to as a single nucleotide mutation. The mutation having the highest frequency in the single nucleotide mutation is called a Single Nucleotide Polymorphism (SNP). The SNPs dotted in the gene are obviously strongly related to many kinds of diseases.

Currently, as a method for detecting the gene mutation including the SNPs, an electrophoresis method is exemplified in which a DNA fragment cut by a restriction enzyme is separated by gel, and then, the DNA fragment is colored and detected by a dye. Though this method is generally used, a problem arises that this method requires a long time for separation or coloring so that it is low in its rapidity.

Further, an integrated substrate for a bio-assembly referred to as what is called a DNA chip in which prescribed DNAs are finely arranged by a micro-array technique begins to be used for detecting a gene mutation. In this DNA chip, since many various kinds of DNA oligonucleotide chains or cDNA or the like are accumulated on a glass substrate or a silicon substrate, many genes can be inspected at one time. The DNA chip is anticipated to be applied to a clinical laboratory field. However, since the DNA chip is based on a method using as a principle the stability of a double-stranded DNA derived from the formation of a mismatch of a nucleobase, it is difficult to control the temperature thereof depending on a base sequence. Further, there is a problem that a pre-process is necessary for modifying an object to be inspected itself by a radioactive material or a fluorescent dye.

Further, in recent years, a real time PCR method in which the object to be inspected is amplified and detected at the same time has been progressively spread as a technique of a rapid quantitative measurement of one stage by a nucleic acid amplification method. However, since a temperature control is complicated in an amplifying reaction, the design of a primer applied to each gene arrangement including the introduction of a probe is complicated, and further, obtained results are frequently different depending on amplifiers or conditions, a problem still remains in view of reproducibility. Further, since a detection is carried out by using the change of a signal with an elapse of time, an operability is undesirably slightly insufficient.

As described above, the usual technique for detecting the gene mutation requires several time-consuming steps such as a precise temperature control or complicated pre-treatments of the object to be inspected, before a measurement. Therefore, in the usual technique, the gene cannot be simply and rapidly inspected.

Thus, the inventors of this application propose a technique, in the document "K. Yoshimoto, S. Nishizawa, M. Minagawa and N. Teramae, "Use of Abasic Site-Containing DNA Strands for Nucleobase Recognition in Water", J. Am. Chem. Soc., 2003, 125, pp. 8982-8983" (refer this document to as a document 1, hereinafter), in which a double-stranded nucleic acid is formed by a single-stranded target DNA having single nucleotide substitution part and a single-stranded probe DNA complementary to this target DNA and having an abasic site (AP site) except a corresponding base corresponding to the single nucleotide substitution part, a receptor molecule having hydrogen bonding characteristics and a fluorescence is added to the double-stranded nucleic acid to form a hydrogen bond to the single nucleotide substitution part, and the change of the fluorescent strength of the receptor molecule is measured to effectively detect the single nucleotide substitution.

Since the technique disclosed in the document 1 does not require, in principle, a complicated operation such as labelling of the target DNA as the object to be inspected or a careful control of temperature, the detection process is quite simple. Further, since the technique does not depend, in principle, on the thermal stability of the double-stranded DNA itself, a rapid detection is possible and reproducibility is also excellent. Further, since a visual recognition using a UV lamp can be realized, the detection can be achieved under a state having no special equipment.

However, in the technique disclosed in the document 1, though a chemical modification that the probe DNA is marked by a fluorescent material is required, a special part such as the abasic site needs to be introduced, which undesirably corresponds to the chemical modification in a strict sense. Further, since the abasic site is introduced, a problem arises that a cost necessary when the probe DNA is synthesized is high.

Kruezer, K.-A., le Coutre, P., Landt, O., Na, I.-K., Schwarz, M., Schultheis, K., Hochhaus, A., and Dörken, B., "Preexistence and evolution of imatinib mesylate-resistant clones in chronic myelogenous leukaemia detected by a PNA-based PCR clamping technique", Ann Hematol (2003), 82, pages 284-289 discloses a technique for the detection of known mutations within the Bcr/Abl kinase domain and involves combining peptide nucleic acid (PNA)-based DNA clamping with a fluorescent hybridization probe assay.

### Disclosure of the Invention

The present invention is proposed by considering the above-described usual circumstances, and it is an object of the present invention to provide a method for detecting a gene mutation and a kit for detecting a gene mutation in which a gene mutation is simply and rapidly detected without performing a chemical modification to a target DNA and a probe DNA.

In order to achieve the above-described object, a method for detecting a gene mutation according to the present invention comprises:
forming a double-stranded nucleic acid from:
   (i) a single-stranded target nucleic acid having a target base composed of one base or two adjacent bases and two kinds of partial sequences that sandwich the target base between them; and
   (ii) two kinds of single-stranded probe oligodeoxynucleotides complementary to the two kinds of partial sequences that sandwich the target base between them;
forming a hydrogen bond by the target base and a receptor by inserting a receptor having hydrogen bonding characteristics into the double-stranded nucleic acid; and identifying the target base on the basis of the bond of the target base and the receptor.

Further, to achieve the above-described object, a kit for detecting a gene mutation according to the present invention comprises: two kinds of single-stranded probe oligodeoxynucleotides complementary to two kinds of partial sequences that sandwich a target base between them in a single-stranded target nucleic acid having the target base composed of one base or two adjacent bases; and a receptor having hydrogen bonding characteristics and inserted into a double-stranded nucleic acid formed by the target nucleic acid and the two kinds of probe oligodeoxynucleotides to form a hydrogen bond with the target base.
In the above-described method for detecting a gene mutation and the kit for detecting a gene mutation, the double-stranded nucleic acid is formed by the target nucleic acid and the two kinds of probe oligodeoxynucleotides to intentionally form a gap site in the double-stranded nucleic acid. The receptor having the hydrogen bonding characteristics, and optionally fluorescence emitting characteristics, is added to the double-stranded nucleic acid to insert the receptor into the gap site and form the hydrogen bond with the target base. When the receptor has fluorescence emitting characteristics, the fluorescent strength of the double-stranded nucleic acid into which the receptor is inserted is measured to detect a gene mutation generated in the target base.

As the above described receptor, candidates are, for instance, a naphthylidine derivative, a quinoline derivative, a pteridine derivative, a coumarin derivative, an indazol derivative, an alloxazine derivative or amiloride.

Here, the receptor may be fixed to a substrate.

That is, to achieve the above-described object, in one embodiment of the method the receptor is fixed to the substrate, and the double-stranded nucleic acid is formed by dropping on the substrate the single-stranded target nucleic acid and the two kinds of single-stranded probe oligodeoxynucleotides.

Further, in order to achieve the above-described object in one embodiment the kit for detecting a gene mutation according to the present invention comprises: two kinds of single-stranded probe oligodeoxynucleotides complementary to two kinds of partial sequences that sandwich a target base between them in a single-stranded target nucleic acid having the target base composed of one base or two adjacent bases; a receptor having hydrogen bonding characteristics and inserted into a double-stranded nucleic acid formed by the target nucleic acid and the two kinds of probe oligodeoxynucleotides to form a hydrogen bond with the target base; and a substrate to which the receptor is fixed.

In the above-described method for detecting a gene mutation and the kit for detecting a gene mutation, the double-stranded nucleic acid is formed by the target nucleic acid and the two kinds of probe oligodeoxynucleotides to intentionally form a gap site in the double-stranded nucleic acid. The double-stranded nucleic acid is dropped on the substrate to which the receptor having the hydrogen bonding characteristics is fixed to insert the receptor into the gap site and form the hydrogen bond with the target base. Then, a gene mutation generated in the target base is detected on the basis of the binding of the receptor to the target base. In this case, when the receptor shows fluorescence emitting characteristics, the target base can be identified on the basis of the change of fluorescent strength of the double-stranded nucleic acid into which the receptor is inserted. Further, the target base can be identified on the basis of the change of a signal strength of a surface plasmon resonance due to the binding of the receptor to the target base or the change of resonance frequency of a crystal oscillator.

Further, one of the two kinds of probe oligodeoxynucleotides may be fixed to the substrate.

That is, to attain the above-described object, in a further embodiment of the method one probe oligodeoxynucleotide of the two kinds of single-stranded probe oligodeoxynucleotides is fixed to a substrate, and the double-stranded nucleic acid is formed by dropping on the substrate the single-stranded target nucleic acid, the other probe oligodeoxynucleotide acid and the receptor.

Further, to achieve the above-described object, in one embodiment the kit for detecting a gene mutation according to the present invention comprises: two kinds of single-stranded probe oligodeoxynucleotides complementary to two kinds of partial sequences that sandwich a target base between them in a single-stranded target nucleic acid having the target base composed of one base or two adjacent bases; a receptor having hydrogen bonding characteristics and inserted into a double-stranded nucleic acid formed by the target nucleic acid and the two kinds of probe oligodeoxynucleotides to form a hydrogen bond with the target base; and a substrate to which one of the two kinds of probe oligodeoxynucleotides is fixed.

In the above-described method for detecting a gene mutation and the kit for detecting a gene mutation, on a substrate to which one probe oligodeoxynucleotide of the two kinds of probe oligodeoxynucleotides is fixed, the target nucleic acid, the other probe oligodeoxynucleotide and the receptor showing the hydrogen bonding characteristics are dropped to form the double-stranded nucleic acid by the target nucleic acid and the two kinds of probe oligodeoxynucleotides and intentionally form a gap site in the double-stranded nucleic acid. The receptor is inserted into the gap site to form the hydrogen bond with the target base. Then, a gene mutation generated in the target base is detected on the basis of the binding of the receptor to the target base. In this case, when the receptor shows fluorescence emitting characteristics, the target base can be identified on the basis of the change of fluorescent strength of the double-stranded nucleic acid into which the receptor is inserted.

Other objects of the present invention and specific advantages obtained by the present invention will be more apparent from the following description of embodiments.

### Brief Description of the Drawings

Fig. 1 is a diagram for explaining a principle for detecting a gene mutation in this embodiment.
Fig. 2 is a diagram for explaining a principle for detection a gene mutation when a receptor molecule is fixed to a substrate.
Fig. 3 is a diagram for explaining a principle for detecting a gene mutation when one of probe oligodeoxynucleotides is fixed to the substrate.
Fig. 4 is a diagram showing fluorescence spectrum after an AMND is added when target bases are guanine and cytosine.
Fig. 5 is a diagram showing fluorescence after the AMND is added when the target bases are guanine and cytosine.
Fig. 6 is a diagram showing a fluorescence quenching effect after the AMND is added when the target bases are guanine, cytosine, adenine and thymine.
Fig. 7 is a diagram showing a fluorescence quenching effect after DiMe-pteridine is added when the target bases are guanine, cytosine, adenine and thymine.
Fig. 8 is a diagram showing a fluorescence quenching effect after amiloride is added when the target bases are guanine, cytosine, adenine and thymine.
Fig. 9 is a diagram showing a fluorescence quenching effect after the AMND is added when the target bases of a PCR amplified target nucleic acid are guanine, cytosine, adenine and thymine.
Fig. 10 is a diagram showing the SPR signal strength of a sensor chip to which an AMND-DPA is fixed when the target bases are guanine, cytosine, adenine and thymine.
Fig. 11 is a diagram showing the SPR signal strength of a sensor chip to which an AcMND-C5A is fixed when the target bases are guanine, cytosine, adenine and thymine.

### Best Mode for Carrying Out the Invention

Ordinarily, the recognition of a nucleobase using a hydrogen bond has a feature that a high base selectivity can be relatively easily obtained by changing the forms or the number of the hydrogen bonds of a receptor molecule. At this time, since a recognizing function based on the formation of the hydrogen bond cannot be anticipated in a completely aqueous solution, most of usual studies are limited under an environment of a nonpolar solvent as in chloroform, which results in, however, the denaturation and precipitation of a nucleic acid in the solvent.

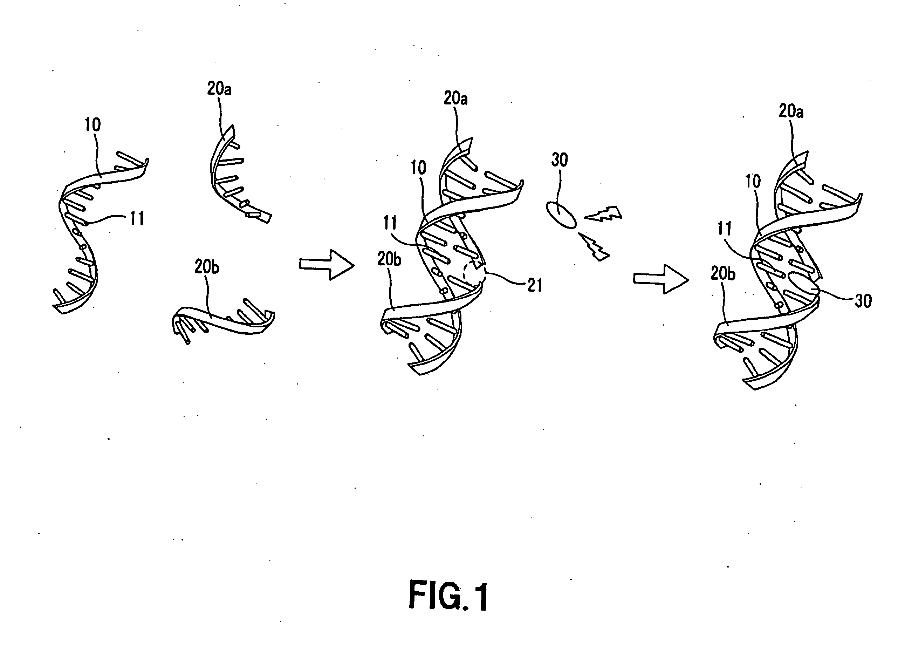

Thus, in this embodiment, as conceptually shown in Fig. 1, solution including a single-stranded target nucleic acid 10 having a target base 11 related to an SNP is mixed with solution including two kinds of single-stranded probe oligodeoxynucleotides 20a and 20b complementary to partial sequences that sandwich the target base 11 between them to hybridize the target nucleic acid 10 with the probe oligodeoxynucleotides 20a and 20b. Thus, a gap site 21 is intentionally formed at a position opposed to the target base 11. Then, a receptor molecule 30 showing hydrogen bonding characteristics is inserted into the gap site 21 as a hydrophobic space to form a hydrogen bond with the target base 11.

As described above, the receptor molecule 30 showing the hydrogen bonding characteristics is inserted into the gap site 21 as the hydrophobic space to form the hydrogen bond with the target base 11. Thus, even in the completely aqueous solution, the nucleobase is effectively recognized, so that the mutation of the target base 11 can be detected.

Further, when the solution including the two kinds of single-stranded probe oligodeoxynucleotides 20a and 20b complementary to the partial sequences that sandwich a plurality of bases of the target nucleic acid 10 between them is employed, the mutation of the plurality of bases can be detected. In this case, when, for instance, the two bases correspond to the gap site 21, two receptor molecules 30 are inserted into the gap site 21.

Here, as the target nucleic acid 10 that can be analyzed in this embodiment, for instance, DNA, cDNA or the like originated from Mammalia including human beings or plants may be exemplified, however, the target nucleic acid is not especially limited to specific nucleic acids and is diluted, concentrated and amplified if necessary.

As the receptor molecule 30 showing the hydrogen bonding characteristics, a reagent having a hydrogen bonding part and showing fluorescence emitting characteristics, a reagent having a hydrogen bonding part and showing fluorescence emitting characteristics is desirable. Specifically, a reagent having a heterocyclic aromatic group is preferable that has at least one stage, or preferably, a plurality of stages of hydrogen bonding parts and can stack on the nucleobase adjacent to the gap part 21. Particularly, a water soluble reagent is preferable. However, in the case of a non-water soluble reagent, this reagent may be met by using a small amount of an organic solvent. As such receptor molecule 30, for instance, may be exemplified a naphthylidine derivative, a quinoline derivative, a pteridine derivative, a coumarin derivative, an indazol derivative, an alloxazine derivative or amiloride.

In the above-described embodiment, a liberated target nucleic acid 10 is allowed to react with liberated detecting nucleic acids 20a and 20b in the solution, however, the present invention is not limited thereto.

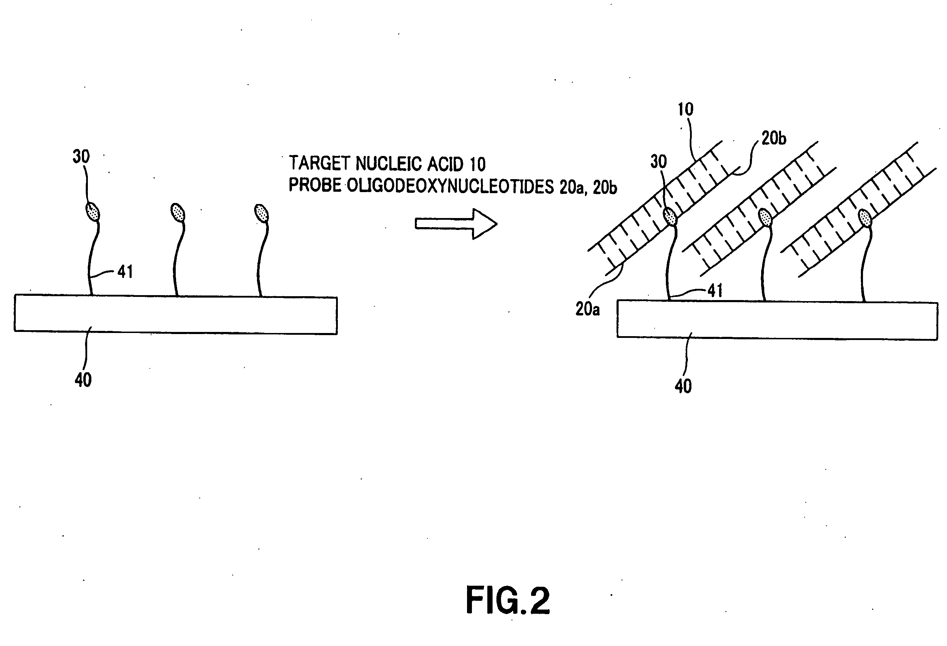

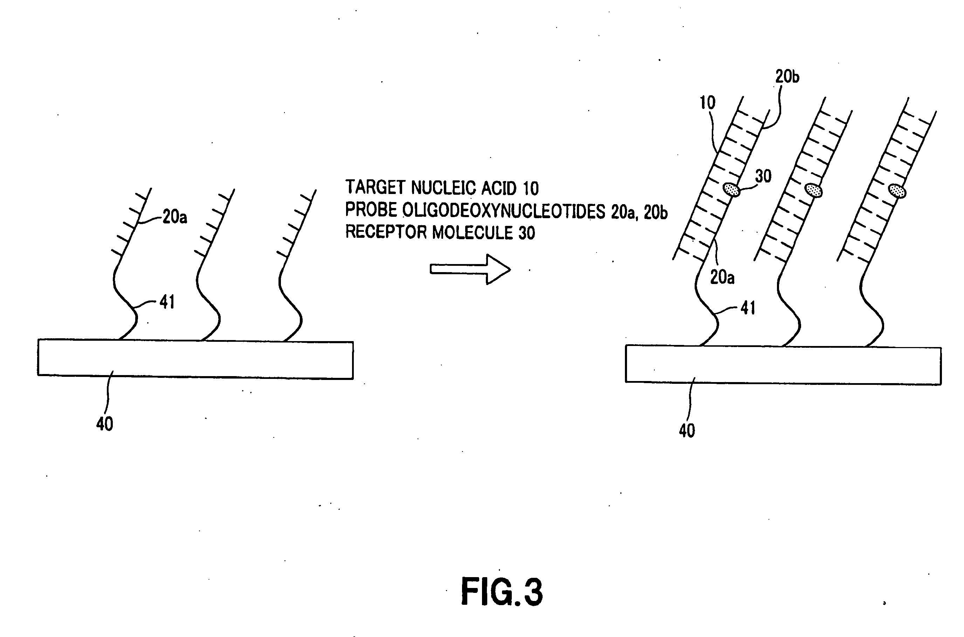

For instance, as schematically shown in Fig. 2, the receptor molecule 30 may be fixed to a substrate 40 through a linker molecule 41 and the solution including the target nucleic acid 10 and the detecting nucleic acids 20a and 20b may be dropped on the substrate 40. Further, as schematically shown in Fig. 3, the detecting nucleic acid 20a may be fixed to the substrate 40 through the linker molecule 41 and the solution including the target nucleic acid 10, the detecting nucleic acid 20b and the receptor molecule 30 may be dropped on the substrate 40.

In such a way, a sensor chip (a micro-array) having many receptor 40 is manufactured and used as a kit for detecting a gene mutation. Thus, the detection with a high throughput that overcomes usual shortcomings can be realized.

In the case of a structure shown in Fig. 2, a gene mutation can be detected by using not the change of a fluorescent strength, but the change of a signal strength of a surface plasmon resonance (SPR) (for instance, see a document "Kazuhiko Nakatani, Shinsuke Sando, and Isao Saito, Nat. Biotechnol., 2001, 19, pp. 51-55", a document "Akio Kobori, Souta Horie, Hitoshi Suda, Isao Saito, and Kazuhiko Nakatani, J. Am. Chem. Soc., 2004, 126, pp. 557-562".). Further, in the case of the structure shown in Fig. 2, the gene mutation may be detected by using the change of resonance frequency of a crystal oscillator.

Now, specific examples of the present invention will be described below in detail by referring to the drawings. However, the present invention is not limited to the following examples and various changes may be made within a scope without departing the gist of the present invention.

### (First Example)

In a first example, as a receptor molecule, 2-amino-7-methyl-1,8-naphthylidine (AMND) as a naphthylidine derivative as shown in a below-described chemical formula was employed. The AMND was synthesized from 2,6-diaminopyridine with reference to a document "E. V. Brown, J. Org. Chem., Vo. 30, pl 607, 1965".

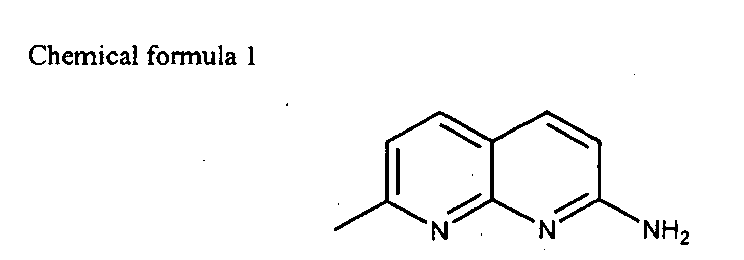

This AMND shows fluorescence emitting characteristics and interacts with a target base when the AMND is inserted into a gap site between two kinds of probe DNAs as described below. Since the fluorescent strength of the AMND changes depending on the difference of the target base, the fluorescent strength is measured so that single nucleotide substitution can be detected. Since the AMND particularly selectively interacts with C (cytosine) as the target base, all single nucleotide substitutions (C/T, C/G, C/A) to which the C (cytosine) is related can be detected.

Here, when the DNA is mixed with the AMND, the AMND may be mixed in the form of solution including the AMND, or may be mixed in the form of powder or a solid. Further, the fluorescence may be visually measured by using a UV lamp or may be measured by using a device such as a fluorescence spectrophotometer, a fluorescence microscope, a densitometer, etc.

In this example, in order to inspect an effect of the detection of the single nucleotide substitution (C/G) by the AMND, a target DNA (a sequence a) of 23 mer and two kinds of probe DNAs (sequences b and c) respectively of 11 mer as described below were prepared as model sequences. Here, in the sequence a, S designates G (guanine) or C (cytosine).
(sequence a) 5'-TCTCCGCACACSTCTCCCCACAC-3' (sequence no. 1)
(sequence b) 5'-GTGTGCGGAGA-3' (sequence no. 2)
(sequence c) 5'-GTGTGGGGAGA-3' (sequence no. 3)

Specifically, in this example, 600 µM target DNA solution (the sequence a) of 25 µl as an object to be inspected, two kinds of 600 µM probe DNA solutions (the sequences b and c) of 25 µl, 500 mM NaCl solution of 50 µl as an ionic strength adjuster, 50 mM sodium cacodylate solution of 50 µl including 5 mM EDTA as a pH buffer and 150 µM AMND solution of 50 µl were mixed together and MilliQ solution was added to the mixed solution to obtain a total quantity of 250 µl. An annealing process was carried out to the obtained DNA solution by a thermal cycler to measure the fluorescent strength. The fluorescence was measured by using a fluorescence measuring cell (quartz cuvette, 2 mm x 10 mm).

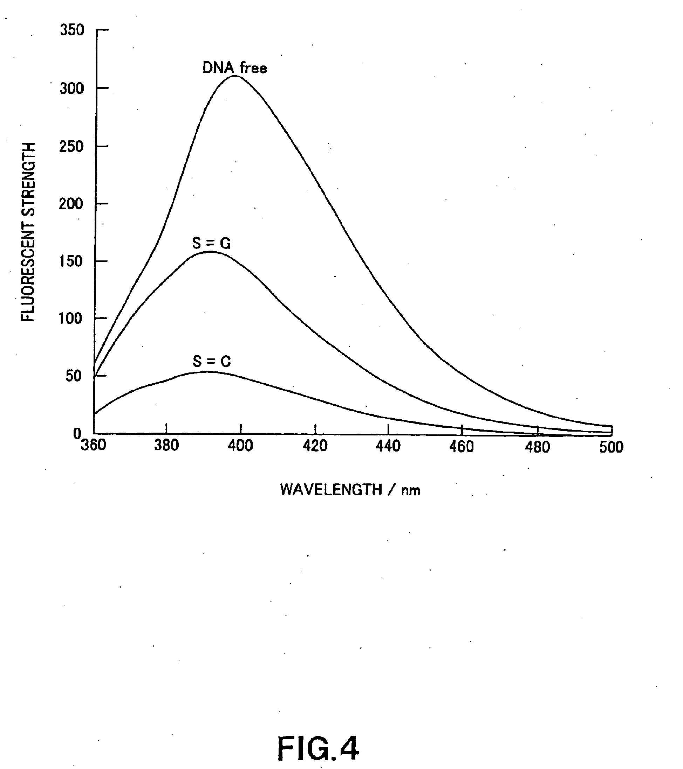

Fig. 4 shows a fluorescence spectrum when the target DNA is not added (DNA free) and fluorescence spectrums when the target base S of the target DNA is G (guanine) and C (cytosine). Here, excitation wavelength in Fig. 4 is 350 nm. As shown in Fig. 4, when the target base S is C (cytosine), the fluorescence is extremely quenched. This phenomenon may be considered to arise, because the AMND is stacked on the nucleobase adjacent the gap site and a stable combined body is formed due to the formation of a hydrogen bond with the target base (C). In such a way, whether or not a quenching exists is detected, so that a user can know that the target base Y is G (guanine) or C (cytosine).

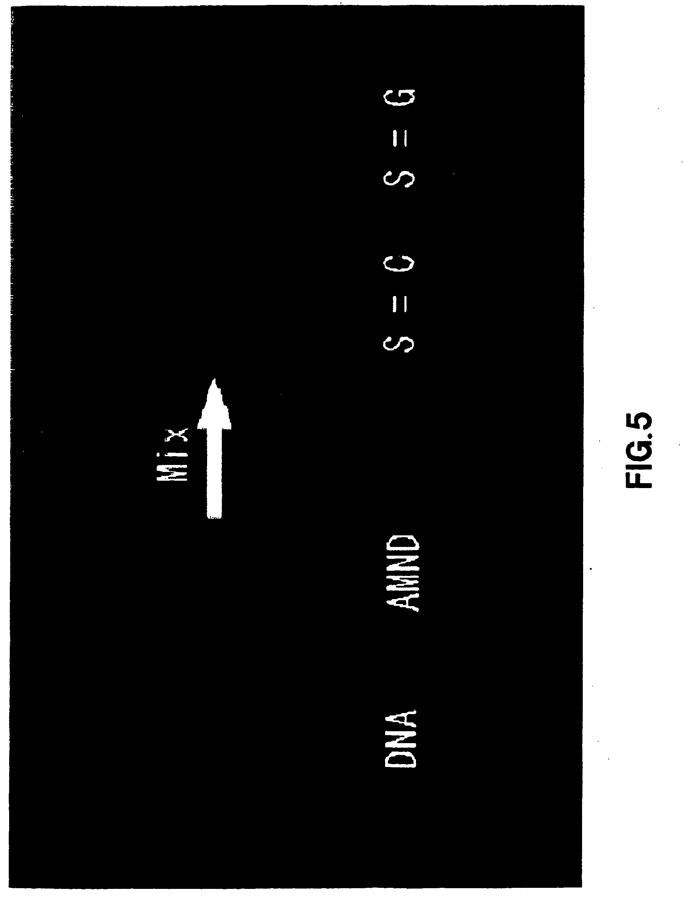

Fig. 5 shows a result obtained when the same DNA solution is put in a transparent tube made of polypropylene and the fluorescence is visually detected by using the UV lamp with an excitation wavelength of 350 nm. In Fig. 5, the fluorescence of the solution including the target DNA and the probe DNAs, and of the solution including the AMND alone is also shown. As shown in Fig. 5, when the target base S is C (cytosine), the fluorescence is extremely quenched and can be even visually recognized.

### (Second Example)

In a second example, the same AMND as that of the first example was used as a receptor molecule to evaluate the adaptability to the detection of all single nucleotide substitutions (C/T, C/G, C/A) to which C (cytosine) is related.

In this example, in order to inspect an effect of the detection of the single nucleotide substitutions (C/T, C/G, C/A) by the AMND, a target DNA (a sequence d) of 107 mer and probe DNAs (sequences e and f) respectively of 15 mer as described below were prepared as model sequences. Here, in the sequence d, N designates G (guanine), C (cytosine), A (adenine) or T (thymine). (sequence e) 5'-TGGCGTAGGCAAGAG-3' (sequence no. 5)
(sequence f) 5' TGGTAGTTGGAGCTG-3' (sequence no. 6)

Specifically, in this example, to 5 µM target single-stranded DNA solution (the sequence d) of 5 µl as an object to be inspected, 5 µM probe DNA solutions (the sequences e and f) of 5 µl were added, and further, 500 mM NaCl solution of 10 µl as an ionic strength adjuster, 50 mM sodium cacodylate solution of 10 µl including 5 mM EDTA as a pH buffer and 5 µM AMND solution of 5 µl were added and MilliQ solution was added to the mixed solution to obtain a total quantity of 50 µl. An annealing process was carried out to the obtained DNA solution by using a thermal cycler to measure the fluorescent strength. The fluorescence was measured by using a fluorescence measuring cell (quartz cuvette, 3 mm x 3 mm).

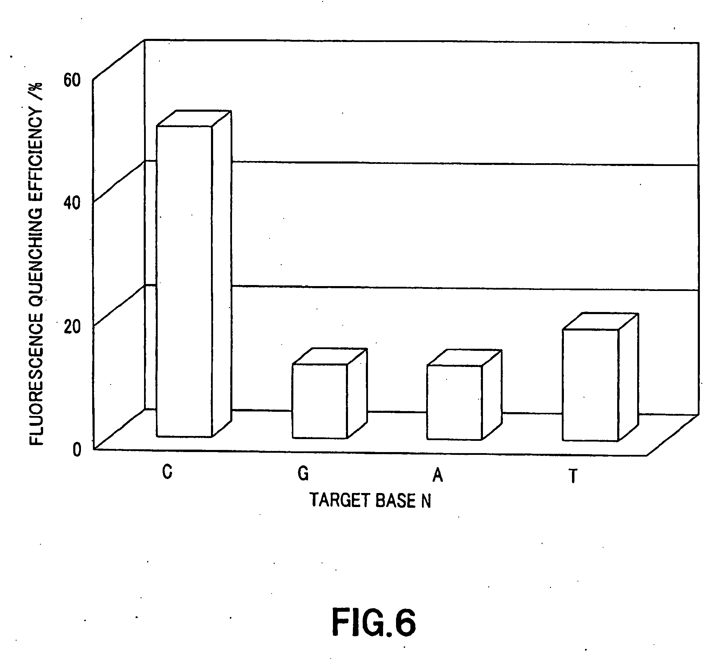

Fig. 6 shows a fluorescence quenching efficiency (%) when the target base N of the target DNA is G (guanine), C (cytosine), A (adenine) or T (thymine). Here, assuming that the fluorescent strength when the target DNA is not present is set to F_{free} and the fluorescent strength when the target DNA is present is set to F_{obs}, the fluorescence quenching efficiency is expressed by ((F_{free}-F_{obs})/F_{free}) × 100. Further, excitation wavelength in Fig. 6 is 350 nm and detected wavelength is 400 nm. As shown in Fig. 6, only when the target base N is C (cytosine), the fluorescence is extremely quenched. In such a way, whether or not a quenching exists is detected so that a user can know whether or not the target base N is C (cytosine). That is, the AMND is used as the receptor molecule so that all the single nucleotide substitutions to which C (cytosine) is related can be detected.

### (Third Example)

In a third example, as a receptor molecule, 2-amino-6,7-dimethyl-4-hydroxypteridine (DiMe-pteridine) as a pteridine derivative as shown in a below-described chemical formula was employed.

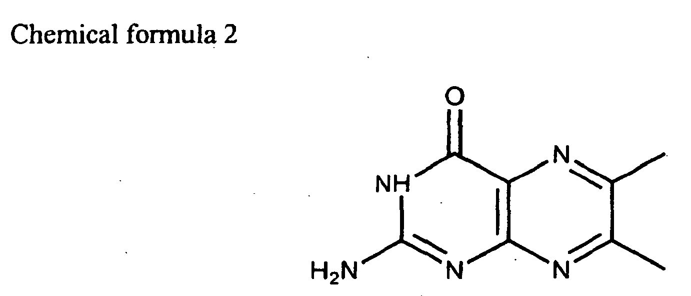

This DiMe-pteridine shows fluorescence emitting characteristics and interacts with a target base when the DiMe-pteridine is inserted into a gap site between two kinds of probe DNAs as described below. Since the fluorescent strength of the DiMe-pteridine changes depending on the difference of the target base, the fluorescent strength is measured so that single nucleotide substitution can be detected. Since the DiMe-pteridine particularly selectively interacts with G (guanine) as the target base, all single nucleotide substitutions (G/C, G/A, G/T) to which the G (guanine) is related can be detected.

In this example, in order to inspect an effect of the detection of the single nucleotide substitution (G/C, G/A, G/T) by the DiMe-pteridine, a target DNA (a sequence g) of 23 mer as described below and the above-described probe DNA (the sequence b) of 11 mer were prepared as model sequences. Here, in the sequence g, N designates G (guanine), C (cytosine), A (adenine) or T (thymine). In the sequence g in this example, since sequences before and after the target base N are the same, one kind of the probe DNA (the sequence b) of two equivalents was added to the target DNA (the sequence g) to form a gap site at a part opposed to the target base N.
(sequence g) 5'-TCTCCGCACACNTCTCCGCACAC-3' (sequence no. 7)

Specifically, in this example, to 5 µM target single-stranded DNA solution (the sequence g) of 10 µl as an object to be inspected, 10 µM probe DNA solution (the sequence b) of 10 µl was added, and further, 500 mM NaCl solution of 10 µl as an ionic strength adjuster, 50 mM sodium cacodylate solution of 10 µl including 5 mM EDTA as a pH buffer and 1 µM DiMe-pteridine solution of 5 µl were added and MilliQ solution was added to the mixed solution to obtain a total quantity of 50 µl. An annealing process was carried out to the obtained DNA solution by using a thermal cycler to measure the fluorescent strength. The fluorescence was measured by using a fluorescence measuring cell (quartz cuvette, 3 mm x 3 mm).

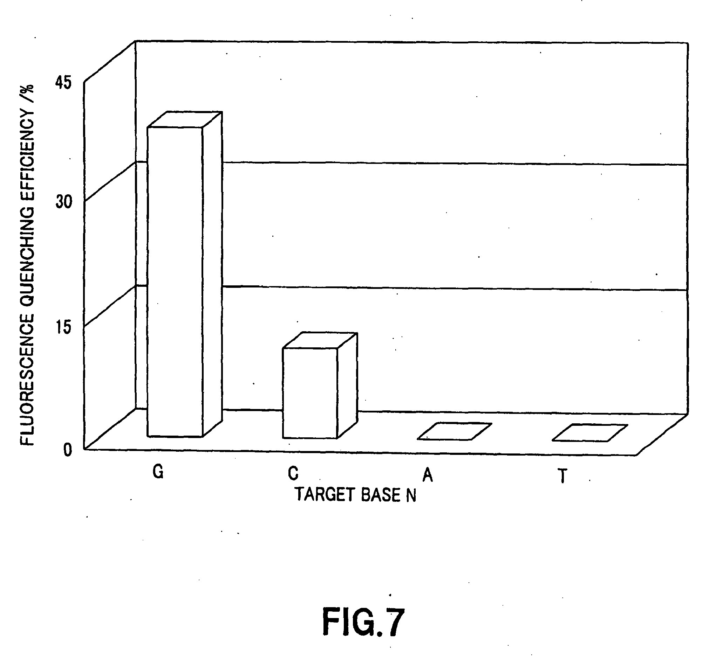

Fig. 7 shows a fluorescence quenching efficiency (%) when the target base N of the target DNA is G (guanine), C (cytosine), A (adenine) or T (thymine). Here, excitation wavelength in Fig. 7 is 343 nm and detected wavelength is 435 nm. As shown in Fig. 7, only when the target base N is G (guanine), the fluorescence is extremely quenched. In such a way, whether or not a quenching exists is detected so that a user can know whether or not the target base N is G (guanine). That is, the DiMe-pteridine is used as the receptor molecule so that all the single nucleotide substitutions to which G (guanine) is related can be detected.

### (Fourth Example)

In a fourth example, as a receptor molecule, amiloride (N-amidino-3,5-diamino-6-chloropyrazinecarboxamide hydrochloride) as shown in a below-described chemical formula was employed.

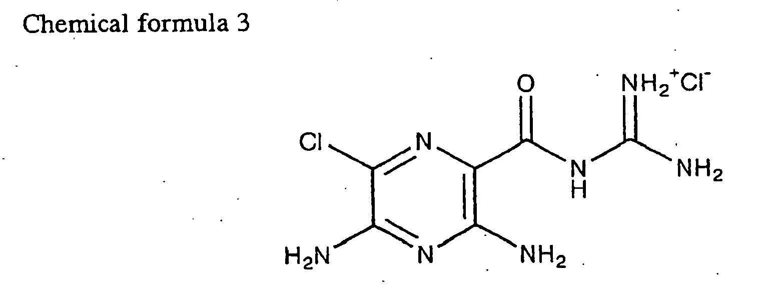

This amyloride shows fluorescence emitting characteristics and interacts with a target base when the amyloride is inserted into a gap part between two kinds of detecting DNAs as described below. Since the fluorescent strength of the amiloride changes depending on the difference of the target base, the fluorescent strength is measured so that single nucleotide substitution can be detected. Since the amiloride particularly selectively interacts with T (thymine) as the target base, all single nucleotide substitutions (T/G, T/C, T/A) to which the T (thymine) is related can be detected.

In this example, in order to inspect an effect of the detection of the single nucleotide substitution (T/G, T/C, T/A) by the amyloride, the above-described target DNA (the sequence g) of 23 mer and the above-described detecting DNA (the sequence b) of 11 mer were prepared as model sequences. Also in this example, the one kind of the detecting DNA (the sequence b) of two equivalents was added to the target DNA (the sequence g) to form a gap part at a part opposed to the target base N.

Specifically, in this example, to 5 µM target single-stranded DNA solution (the sequence g) of 10 µl as an object to be inspected, 10 µM detecting DNA solution (the sequence b) of 10 µl was added, and further, 500 mM NaCl solution of 10 µl as an ionic strength conditioner, 50 mM sodium cacodylate solution of 10 µl including 5 mM EDTA as a pH buffer and 1 µM amyloride solution of 5 µl were added and MilliQ solution was added to the mixed solution to obtain a total quantity of 50 µl. An annealing process was carried out to the obtained DNA solution by using a thermal cycler to measure the fluorescent strength. The fluorescence was measured by using a fluorescence measuring cell having an optical path length of 3 mm × 3 mm.

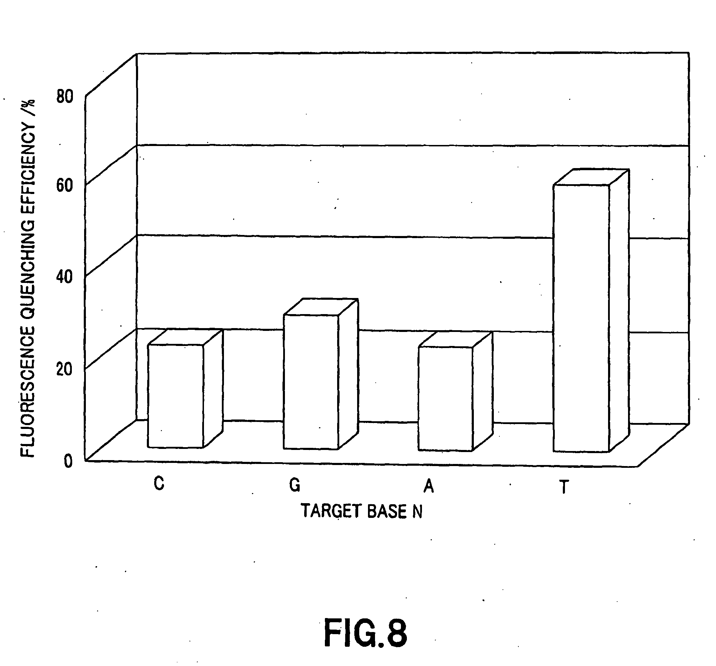

Fig. 8 shows a fluorescence quenching efficiency (%) when the target base N of the target DNA is G (guanine), C (cytosine), A (adenine) or T (thymine). Here, excitation wavelength in Fig. 8 is 361 nm and detected wavelength is 415 nm. As shown in Fig. 8, only when the target base N is T (thymine), the fluorescence is extremely quenched. In such a way, whether or not a quenching exists is detected so that a user can know whether or not the target base N is T (guanine). That is, the amiloride is used as the receptor molecule so that all the single nucleotide substitutions to which T (thymine) is related can be detected.

### (Fifth Example)

In a fifth example, the same AMND as that of the first example was used as a receptor molecule to evaluate the adaptability to a PCR product.

In this example, in order to inspect the adaptability to the PCR product by the AMND, the above-described target DNA (the sequence d) of 107 mer and the above-described probe DNAs (the sequences e and f) respectively of 15 mer were prepared as model sequences.

Here, the target DNA (the sequence d) in this example amplifies its antisense strand by a below-described forward primer (a sequence h) and a reverse primer (a sequence i) in an area including a codon 12 of a K-ras gene.
(sequence h) 5'-GACTGAATATAAACTTGTGG-3' (sequence no. 8)
(sequence i) 5'-CTATTGTTGGATCATATTCG-3' (sequence no. 9)

PCR solution was prepared with reference to a protocol of TaKaRa Taq (produced by Takara Bio Inc.). PCR reaction solution has the following composition.

| | |
|---|---|
| Forward primer | 20 pmol (final concentration of 0.2 µM) |
| Reverse primer | 300 pmol (final concentration of 3.0 µM) |
| Target DNA (the sequence d) | 0.5 ng |
| TaKaRa Taq (DNA polymelase) | 2.5 U |
| 10 × PCR buffer | 10 µl |
| 2.5 mM dNTP | 8 µl |

These materials were mixed together in a 0.2 ml PCR tube, and further, MilliQ solution processed by an autoclave was added to the mixed solution to obtain a total quantity of 100 µl. Then, a PCR reaction was carried out in accordance with a protocol that the mixed solution was cooled to 4°C via processes carried out at 94°C for 5 minutes (94°C for 30 seconds to 52°C for 30 seconds to 72°C for 30 seconds) × 40 cycles to 72°C for 7 minutes. Thus, the target DNA (the sequence d) was amplified.

After the above-described PCR reaction was carried out, to the PCR reaction solution of 40 µl, a pH buffer (2 M sodium cacodylate, 33 mM EDTA, pH = 7.0) of 2.5 µl, 100 µM probe DNA solution (the sequences e and f) respectively of 2.5 µl and 1 µM AMND solution of 5 µl were added to obtain a total quantity of 50 µl. The fluorescent strength of the obtained DNA solution was measured by using a fluorescence measuring cell (quartz cuvette, 3 mm x 3 mm). A measuring temperature is 5°C.

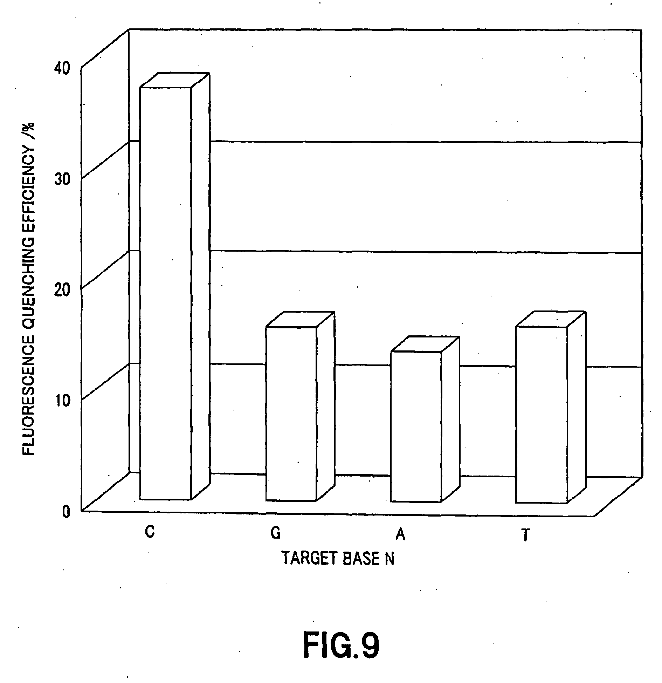

Fig. 9 shows a fluorescence quenching efficiency (%) when the target base N of the target DNA is G (guanine), C (cytosine), A (adenine) or T (thymine). Here, excitation wavelength in Fig. 9 is 350 nm and detected wavelength is 400 nm. As shown in Fig. 9, only when the target base N is C (cytosine), the fluorescence is extremely quenched. In such a way, whether or not a quenching exists is detected so that a user can know whether or not the target base N is C (cytosine). Further, in this example, since an operation for removing DNA polymelase or dNTP or an accurate temperature control is not required, the PCR product can be rapidly and simply analyzed.

### (Sixth Example)

In a sixth example, the detection of single nucleotide substitution by a surface plasmon resonance (SPR) method was evaluated. As a receptor molecule, AMND-DPA (N- (3-Amino-propyl) -N'- (7-methyl- [1,8] naphthyridin-2-yl) propane-1,3-diamine) as shown in a below-described chemical formula was used to form a sensor chip (a micro-array) having the AMND-DPA fixed to a metal substrate.

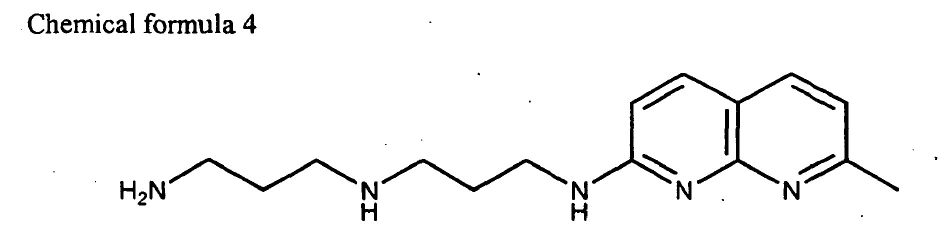

In the AMND-DPA, an alkyl chain having an amino group at its terminal end is introduced to a mother skeleton of the AMND to fix the AMND-DPA on the metal substrate and the AMND-DPA is synthesized from 2,6-diaminopyridine. When the AMND-DPA is inserted into a gap site between two kinds of probe DNAs on the metal substrate, the AMND-DPA particularly selectively interacts with C (cytosine). At this time, since the signal strength of the SPR changes depending on the difference of the target base, the signal strength is measured so that all single nucleotide substitutions (C/T, C/G, C/A) to which the C (cytosine) is related can be detected.

In this example, in order to inspect an effect of the detection of the single nucleotide substitution by the sensor chip to which the AMND-DPA is fixed, the above-described target DNA (the sequence g) of 23 mer and the above-described probe DNA (the sequence b) of 11 mer were prepared as model sequences. Also in this example, the one kind of the probe DNA (the sequence b) of two equivalents was added to the target DNA (the sequence g) to form a gap site at a part opposed to the target base N.

Specifically, in this example, to 25 µM target DNA solution (the sequence g) of 10 µl as an object to be inspected, 20 µM probe DNA solution (the sequence b) of 20 µl was added, and further, PBS-EP buffer (0.67 M phosphoric acid buffer solution, 1.5 M NaCI, 3 mM EDTA, 0.005 % Surfactant P20, pH = 6.4) was added thereto to obtain a total quantity of 500 µl. Further, an annealing process was carried out to the obtained DNA solution by using a thermal cycler.

Further, as the sensor chip, a sensor chip CM5 (produced by Biacore AB) was used. The AMND-DPA was fixed to the sensor chip by using an amine coupling kit (produced by Biacore AB). Specifically, NHS (N-hydroxysuccinimide)/EDC (N-ethyl-N'-(3-dimethylaminoprpyl) carbodiimide hydrochloride) aqueous solution of 50 µl was injected to the sensor chip CM5 to activate a carboxyl group on the surface of the sensor chip by the NHS. Subsequently, AMND-DPA solution (diluted with 10 mM acetic acid buffer solution, pH = 5.5) of 0.20 mg/ml (0.73 mM) was injected to fix the AMND-DPA on the substrate. After that, 1 M ethanol amine aqueous solution of 50 µl was injected to block a remaining active NHS group. Further, the substrate was cleaned by using 8 mM NaOH aqueous solution of 60 µl.

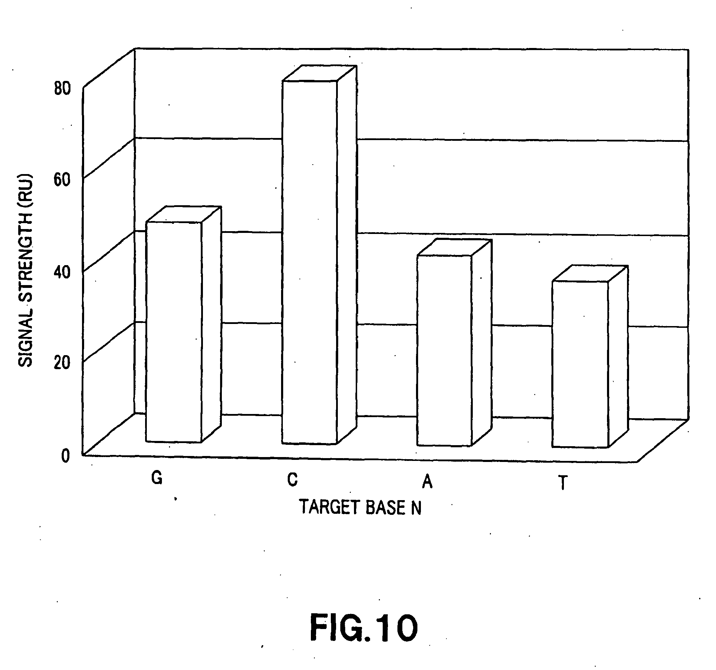

Fig. 10 shows the signal strength (RU: Response Unit) of the SPR when the target base N of the target DNA is G (guanine), C (cytosine), A (adenine) or T (thymine). Here, an amount of the DNA solution used for measuring the SPR was 90 µl and the DNA solution was injected at a flow rate of 30 µl/minute. Fig. 10 shows the signal strength of the SPR after 180 seconds subsequent to the injection. A quantity of fixed AMND-DPA is about 0.20 ng/mm² and a measuring temperature is 5°C.

As shown in Fig. 10, when the target base N is C (cytosine), the signal strength of the SPR is the largest. This phenomenon may be considered to arise, because the AMND-DPA is stacked on the nucleobase adjacent to the gap site and a stable complex is formed due to the formation of a hydrogen bond with the target base (C), and consequently, a dielectric constant in the vicinity of the surface of the metal substrate changes. In such a way, the signal strength of the SPR is detected to know whether or not the target base N is C (cytosine). That is, the sensor chip to which the AMND-DPA is fixed is used so that all the single nucleotide substitutions to which the C (cytosine) is related can be detected.

### (Seventh Example)

In a seventh example, the detection of single nucleotide substitution by a surface plasmon resonance (SPR) method was evaluated like the sixth example. As a receptor molecule, AcMND-C5A (6-Amino-hexanoic acid (7-methyl-[1,8]naphthyridin-2-yl)-amide) as shown in a below-described chemical formula was used to form a sensor chip having the AcMND-C5A fixed to a metal substrate.

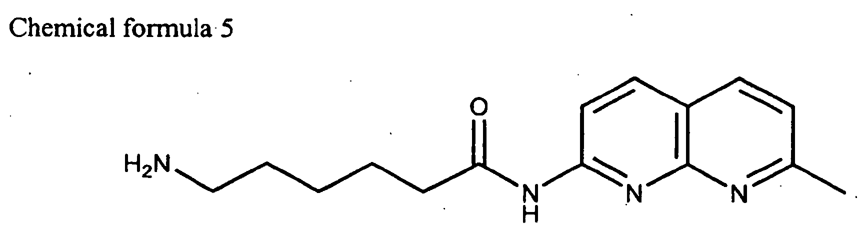

In the AcMND-C5A, an alkyl chain having an amino group at its terminal end is introduced to a mother skeleton of an AMND to fix the AcMND-C5A on the metal substrate and the AcMND-C5A is synthesized from 2,6-diaminopyridine. When the AcMND-C5A is inserted into a gap site between two kinds of probe DNAs on the metal substrate, the AcMND-C5A particularly selectively interacts with G (guanine). At this time, since the signal strength of the SPR changes depending on the difference of the target base, the signal strength is measured so that all single nucleotide substitutions (G/C, G/A, G/T) to which the G (guanine) is related can be detected.

In this example, in order to inspect an effect of the detection of the single nucleotide substitution by the sensor chip to which the AcMND-C5A is fixed, the above-described target DNA (the sequence g) of 23 mer and the above-described probe DNA (the sequence b) of 11 mer were prepared as model sequences. Also in this example, the one kind of the probe DNA (the sequence b) of two equivalents was added to the target DNA (the sequence g) to form a gap site at a part opposed to the target base N.

Specifically, in this example, to 200 µM target DNA solution (the sequence g) of 5 µl as an object to be inspected, 400 µM probe DNA solution (the sequence b) of 5 µl was added, and further, PBS-EP buffer (0.67 M phosphoric acid buffer solution, 1.5 M NaCl, 3 mM EDTA, 0.005 % Surfactant P20, pH = 6.4) was added thereto to obtain a total quantity of 500 µl. Further, an annealing process was carried out to the obtained DNA solution by using a thermal cycler.

Further, as the sensor chip, a sensor chip CM5 (produced by Biacore AB) was used. The AcMND-C5A was fixed to the sensor chip by using an amine coupling kit (produced by Biacore AB). Specifically, NHS/EDC aqueous solution of 50 µl was injected to the sensor chip CM5 to activate a carboxyl group on the surface of the sensor chip by the NHS. Subsequently, AcMND-C5A solution (diluted with 10 mM acetic acid buffer solution, pH = 5.5) of 1.0 mg/ml (3.7 mM) was injected to fix the AcMND-C5A on the substrate. After that, 1 M ethanol amine aqueous solution of 50 µl was injected to block a remaining active NHS group. Further, the substrate was cleaned by using 8 mM NaOH aqueous solution of 60 µl.

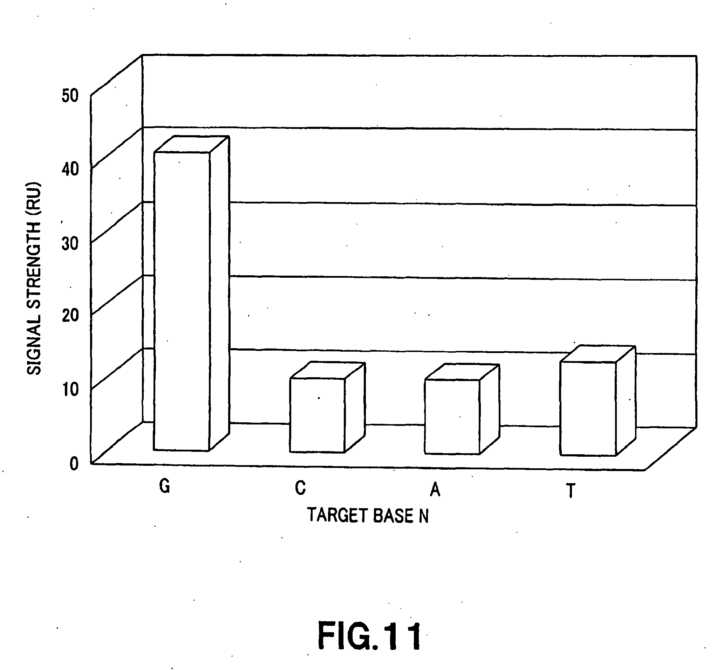

Fig. 11 shows the signal strength (RU: Response Unit) of the SPR when the target base N of the target DNA is G (guanine), C (cytosine), A (adenine) or T (thymine). Here, an amount of the DNA solution used for measuring the SPR was 60 µl and the DNA solution was injected at a flow rate of 20 µl/minute. Fig. 11 shows the signal strength of the SPR after 180 seconds subsequent to the injection. A quantity of fixed AcMND-C5A is about 1.6 ng/mm² and a measuring temperature is 5°C.

As shown in Fig. 11, when the target base N is G (guanine), the signal strength of the SPR is the largest. This phenomenon may be considered to arise, because the AcMND-C5A is stacked on the nucleobase adjacent to the gap site and a stable complex is formed due to the formation of a hydrogen bond with the target base (G), and consequently, a dielectric constant in the vicinity of the surface of the metal substrate changes. In such a way, the signal strength of the SPR is detected to know whether or not the target base N is G (guanine). That is, the sensor chip to which the AcMND-C5A is fixed is used so that all the single nucleotide substitutions to which the G (guanine) is related can be detected.

As can be understood from the specific examples, according to the method for detecting a gene mutation in this embodiment, the double-stranded nucleic acid is formed by the single-stranded target nucleic acid 10 having the target base 11 composed of one or more continuous bases and the two kinds of single-stranded probe oligodeoxynucleotides 20a and 20b complementary to two kinds of partial sequences that sandwich the target base 11 between them. The receptor molecule 30 having the hydrogen bonding characteristics, and optionally fluorescence emitting characteristics, is added to the double-stranded nucleic acid to form the hydrogen bond with the target base 11. When the receptor has fluorescence emitting characteristics, the fluorescent strength of the double-stranded nucleic acid bonded with the receptor molecule 30 is measured so that the gene mutation such as the single nucleotide substitution can be effectively detected.

Since a complicated operation such as labelling of the target DNA 10 as the object to be inspected or a heat control is not especially required, the detection process is quite simple. Further, since the method for detecting a gene mutation does not depend, in principle, on the thermal stability of the double-stranded DNA itself, a rapid detection is possible and reproducibility is also excellent. Further, since a visual recognition using a UV lamp can be realized, the detection can be achieved under a state having no special equipment.

Further, the sensor chip (a micro-array) having many receptor molecules 30 or the probe oligodeoxynucleotides 20a accumulated on the substrate is manufactured and used as a kit for detecting a gene mutation. Thus, the detection with a high throughput that overcomes usual shortcomings can be realized.

In the above-described embodiment, the two kinds of probe oligodeoxynucleotides complementary to the two kinds of partial sequences that sandwich the target base between them are hybridized with the target nucleic acid to intentionally introduce the gap site. It is also known that the gap site is also produced during a restoring process of the DNA. (see a document "Erling Seeberg, Lars Eide and Magnar Bjoras, Trend. Biochem. Sci., 1995, 20(10), pp. 391-397".) Thus, as shown in Fig. 2, the double-stranded DNA solution can be dropped on the substrate to which the receptor molecule is fixed to react with the receptor molecule to detect whether or not the DNA is damaged.

### Industrial Applicability

As described above, according to the present invention, the double-stranded nucleic acid is formed by the target nucleic acid and the two kinds of probe oligodeoxynucleotides to form a gap site at a position opposed to the target base. The hydrogen bond is formed by the receptor inserted into the gap site and the target base so that a gene mutation such as the single nucleotide substitution generated in the target base can be effectively detected.

Further, the receptor or one of the two kinds of probe oligodeoxynucleotides may be fixed to a substrate and the obtained material is used as a kit for detecting a gene mutation. Thus, the detection with a high throughput that overcomes the usual disadvantages can be realized.

## Claims

1. A method for detecting a gene mutation comprising:
forming a double-stranded nucleic acid from:
(i) a single-stranded target nucleic acid having a target base composed of one base or two adjacent bases and two kinds of partial sequences that sandwich the target base between them; and
(ii) two kinds of single-stranded probe oligodeoxynucleotides complementary to the two kinds of partial sequences that sandwich the target base between them;
forming a hydrogen bond by the target base and a receptor by inserting a receptor having hydrogen bonding characteristics into the double-stranded nucleic acid; and identifying the target base on the basis of the bond of the target base and the receptor.

2. The method for detecting a gene mutation according to claim 1, wherein the receptor has a heterocyclic aromatic group and is stabilised by the formation of a hydrogen bond to the target base and a stacking interaction with the base adjacent to the receptor to form a pair with the target base.

3. The method for detecting a gene mutation according to claim 2, wherein the receptor is at least one of a naphthylidine derivative, a quinoline derivative, a pteridine derivative, a coumarin derivative, an indazol derivative, an alloxazine derivative and amiloride.

4. The method for detecting a gene mutation according to claim 1, wherein the receptor is fixed to a substrate,
the double-stranded nucleic acid is formed by dropping on the substrate the single-stranded target nucleic acid and the two kinds of single-stranded probe oligodeoxynucleotides.

5. The method for detecting a gene mutation according to claim 4, wherein the target base is identified on the basis of the change of a signal strength of a surface plasmon resonance due to the binding of the receptor to the target base.

6. The method for detecting a gene mutation according to claim 1, wherein
one probe oligodeoxynucleotide of the two kinds of single-stranded probe oligodeoxynucleotides is fixed to a substrate; and
the double-stranded nucleic acid is formed by dropping on the substrate the single-stranded target nucleic acid, the other probe oligodeoxynucleotide and the receptor.

7. The method for detecting a gene mutation according to any one of claims 1, 4 and 6, wherein the receptor shows fluorescence emitting characteristics and the target base is identified on the basis of the change of fluorescence strength of the double-stranded nucleic acid into which the receptor is inserted.

8. A kit suitable for the method of claim 1, comprising:
two kinds of single-stranded probe oligodeoxynucleotides complementary to two kinds of partial sequences that sandwich a target base between them in a single-stranded target nucleic acid having the target base composed of one base or two adjacent bases;
a receptor having hydrogen bonding characteristics and inserted into a double-stranded nucleic acid formed by the single-stranded target nucleic acid and two kinds of the probe oligodeoxynucleotides to form a hydrogen bond with the target base.

9. The kit for detecting a gene mutation according to claim 8, further comprising a substrate to which the receptor is fixed.

10. The kit for detecting a gene mutation according to claim 8, further comprising a substrate to which one detecting nucleic acid of the two kinds of single stranded probe oligodeoxynucleotides is fixed.

11. The kit for detecting a gene mutation according to any one of claims 8 to 10, wherein the receptor shows fluorescence emitting characteristics.

## Patentansprüche

1. Verfahren zum Erfassen einer Genmutation, bei dem man:
eine doppelstrangige Nukleinsäure ausbildet aus:
(i) einer einzelstrangigen Zielnukleinsäure, die eine Zielbase aufweist, die aus einer Base oder aus zwei benachbarten Basen und zwei Arten von Teilsequenzen, die die Zielbase zwischen sich sandwichartig einschließen, zusammengesetzt ist, und
(ii) zwei Arten von einzelstrangigen Sondenoligodesoxynukleotiden, die zu den zwei Arten von Teilsequenzen, die die Zielbase zwischen sich sandwichartig einschließen, komplementär sind,
eine Wasserstoffbindung zwischen der Zielbase und einem Rezeptor ausbildet, indem man einen Rezeptor mit Wasserstoffbindungseigenschaften in die zweistrangige Nukleinsäure inseriert, und die Zielbase auf der Grundlage der Bindung der Zielbase und des Rezeptors identifiziert.

2. Verfahren zum Erfassen einer Genmutation nach Anspruch 1, wobei der Rezeptor eine heterozyklische aromatische Gruppe aufweist und durch die Ausbildung einer Wasserstoffbindung mit der Zielbase und durch eine Stapelwechselwirkung mit der zu dem Rezeptor benachbarten Base unter Ausbildung eines Paares mit der Zielbase stabilisiert wird.

3. Verfahren zum Erfassen einer Genmutation nach Anspruch 2, wobei der Rezeptor wenigstens eines unter einem Naphthylidinderivat, einem Chinolinderivat, einem Ptelidinderivat, einem Cumarinderivat, einem Indazolderivat, einem Alloxazinderivat und Amilorid ist.

4. Verfahren zum Erfassen einer Genmutation nach Anspruch 1, wobei der Rezeptor auf einem Substrat fixiert ist,
wobei die doppeistrangige Nukleinsäure ausgebildet wird durch das Auftropfen der einzeistangigen Zielnukleinsäure und der zwei Arten von einzelstrangigen Sondenoligodesoxynukleotiden auf das Substrat.

5. Verfahren zum Erfassen einer Genmutation nach Anspruch 4, wobei die Zielbase auf der Grundlage der Veränderung einer Signalstärke einer Oberflächenplasmonresonanz infolge der Bindung des Rezeptors an die Zielbase identifiziert wird.

6. Verfahren zum Erfassen einer Genmutation nach Anspruch 1, wobei
ein Sondenaligodesoxynukleotid der zwei Arten von einzelstrangigen Sondenoligodesoxynuldeotiden auf einem Substrat fixiert wird und
die doppeistrangige Nukleinsäure ausgebildet wird durch das Auftropfen der einzelstrangigen Zielnukleinsäure, des anderen Sondenoligodesoxynukleotids und des Rozeptors auf das Substrat.

7. Verfahren zum Erfassen einer Genmutation nach einem der Anspruche 1, 4 und 6, wobei der Rezeptor fluoreszenzemittierende Eigenschaften aufzeigt und die Zielbase auf der Grundlage der Veränderung der Fluoreszenzstärke der doppeistrangigen Nukleinsäure, in die der Rezeptor inseriert ist, identifiziert Wird.

8. Kit, welches für das Verfahren nach Anspruch 1 geeignet ist, umfassend:
zwei Arten von einzelstrangigen Sondenoligodesoxynukleotiden, die mit zwei Arten von Teilsequenzen, die eine Zielbase in einer einzeistrangigen Zieinukleinsäure mit der Zielbase, die aus einer Base oder zwei benachbarten Basen zusammengesetzt ist, zwischen sich sandwichartig einschließen, komplementär sind,
einen Rezeptor mit Wasserstoffbindungseigenschaften und inseriert in eine doppelstrangige Nukleinsäure, die gebildet wird durch die einzelstrangige Zielnukleinsäure und die zwei Arten der Sondenoligodesoxynukleotide, um eine Wasserstoffbindung mit der Zielbase auszubilden.

9. Kit für die Erfassung einer Genmutation nach Anspruch 8, welches weiterhin ein Substrat umfasst, auf welchen der Rezeptor fixiert ist.

10. Kit zum Erfassen einer Genmutation nach Anspruch 8, welches weiterhin ein Substrat umfasst, auf welches eine Detektionsnukleinsäure der zwei Arten von einzeistrangigen Sondenoligodesoxynukleotiden fixiert ist.

11. Kit zum Erfassen einer Genmutation nach einem der Ansprüche 8 bis 10, wobei der Rezeptor fluoreszenzemittierende Eigenschaften aufzeigt.

## Revendications

1. Procédé pour détecter une mutation d'un gène, consistant à :
former un acide nucléique double brin à partir de :
(i) un acide nucléique cible simple brin comportant une base cible composée d'une base ou de deux bases adjacentes, et deux types de séquences partielles où la base cible est prise en sandwich entre celles-ci ; et
(ii) deux types d'oligodésoxynucléotides sonde simple brin complémentaires aux deux types de séquences partielles où la base cible est prise en sandwich entre celles-ci ;
former une liaison hydrogène par la base cible et un récepteur en insérant un récepteur ayant des caractéristiques de liaison à l'hydrogène dans l'acide nucléique double brin ; et
identifier la base cible en se basant sur la liaison de la base cible et du récepteur.

2. Procédé pour détecter une mutation d'un gène selon la revendication 1, dans lequel le récepteur possède un groupe aromatique hétérocyclique et est stabilisé par la formation d'une liaison hydrogène avec la base cible et une interaction d'empilement avec la base adjacente au récepteur afin de former une paire avec la base cible.

3. Procédé pour détecter une mutation d'un gène selon la revendication 2, dans lequel le récepteur est au moins l'un parmi un dérivé de la naphtylidine, un dérivé de la quinoline, un dérivé de la ptéridine, un dérivé de la coumarine, un dérivé de l'indazole, un dérivé de l'alloxazine et un amiloride.

4. Procédé pour détecter une mutation d'un gène selon la revendication 1, dans lequel le récepteur est fixé à un substrat,
l'acide nucléique double brin est formé en déposant sur le substrat l'acide nucléique cible simple brin et les deux types d'oligodésoxynucléotides sonde simple brin.

5. Procédé pour détecter une mutation d'un gène selon la revendication 4, dans lequel la base cible est identifiée en se basant sur la modification de l'intensité d'un signal d'une résonance plasmonique de surface due à la liaison du récepteur à la base cible.

6. Procédé pour détecter une mutation d'un gène selon la revendication 1, dans lequel
un oligodésoxynucléotide sonde des deux types d'oligodésoxynucléotides sonde simple brin est fixé à un substrat ; et
l'acide nucléique double brin est formé en déposant sur le substrat l'acide nucléique cible simple brin, l'autre oligodésoxynucléotide sonde et le récepteur.

7. Procédé pour détecter une mutation d'un gène selon l'une quelconque des revendications 1, 4 et 6, dans lequel le récepteur montre des caractéristiques d'émission d'une fluorescence et la base cible est identifiée en se basant sur la modification de l'intensité de la fluorescence de l'acide nucléique double brin dans lequel est inséré le récepteur.

8. Kit approprié pour le procédé selon la revendication 1, comprenant :
deux types d'oligodésoxynucléotides sonde complémentaires à deux types de séquences partielles, où une base cible est prise en sandwich entre celles-ci, dans un acide nucléique cible simple brin comportant la base cible composée d'une base ou de deux bases adjacentes ;
un récepteur ayant des caractéristiques de liaison à l'hydrogène et qui est inséré dans un acide nucléique double brin formé par l'acide nucléique cible simple brin et les deux types d'oligodésoxynucléotides sonde afin de former une liaison hydrogène avec la base cible.

9. Kit pour détecter une mutation d'un gène selon la revendication 8, comprenant en outre un substrat auquel est fixé le récepteur.

10. Kit pour détecter une mutation d'un gène selon la revendication 8, comprenant en outre un substrat auquel un acide nucléique de détection des deux types d'oligodésoxynucléotides sonde simple brin est fixé.

11. Kit pour détecter une mutation d'un gène selon l'une quelconque des revendications 8 à 10, dans lequel le récepteur montre des caractéristiques d'émission d'une fluorescence.


### SEQUENCE LISTING

<110> Japan Science and Technology Agency
<120> Method of detecting gene mutation and kit for detecting gene
   mutation
<130> P026834EP
<140> 05727696.6
   <141> 2005-03-18
<150> JP 2004-080703
   <151> 2004-03-19
<160> 9
<170> PatentIn version 3.3
<210> 1
   <211> 23
   <212> DNA
   <213> Artificial
<220>
   <223> Artificially synthesized sequence
<400> 1
   tctccgcaca cstctcccca cac 23
<210> 2
   <211> 11
   <212> DNA
   <213> Artificial
<220>
   <223> Artificially synthesized sequence
<400> 2
   gtgtgcggag a 11
<210> 3
   <211> 11
   <212> DNA
   <213> Artificial
<220>
   <223> Artificially synthesized sequence
<400> 3
   gtgtggggag a 11
<210> 4
   <211> 107
   <212> DNA
   <213> Artificial
<220>
   <223> Artificially synthesized sequence
<220>
   <221> misc_feature
   <222> (75)..(75)
   <223> n is a, c, g, or t
<400> 4
   ctattgttgg atcatattcg tccacaaaat gattctgaat tagctgtatc gtcaaggcac 60
   tcttgcctac gccancagct ccaactacca caagtttata ttcagtc 107
<210> 5
   <211> 15
   <212> DNA
   <213> Artificial
<220>
   <223> Artificially synthesized sequence
<400> 5
   tggcgtaggc aagag 15
<210> 6
   <211> 15
   <212> DNA
   <213> Artificial
<220>
   <223> Artificially synthesized sequence
<400> 6
   tggtagttgg agctg 15
<210> 7
   <211> 23
   <212> DNA
   <213> Artificial
<220>
   <223> Artificially synthesized sequence
<220>
   <221> misc_feature
   <222> (12)..(12)
   <223> n is a, c, g, or t
<400> 7
   tctccgcaca cntctccgca cac 23
<210> 8
   <211> 20
   <212> DNA
   <213> Artificial
<220>
   <223> Artificially synthesized primer sequence
<400> 8
   gactgaatat aaacttgtgg 20
<210> 9
   <211> 20
   <212> DNA
   <213> Artificial
<220>
   <223> Artificially synthesized primer sequence
<400> 9
   ctattgttgg atcatattcg 20